# EUROPÄISCHE PATENTSCHRIFT

(11) **EP 1 683 543 B1**
(45) Veröffentlichungstag und Bekanntmachung des Hinweises auf die Patenterteilung: **15.10.2008**
(21) Anmeldenummer: 05001047.9
(22) Anmeldetag: 19.01.2005
(51) Int. Cl.: A61M 39/10

(54) **Kanülenansatzsystem**
System for cannula hub and base
Système d'embase pour canule

(43) Veröffentlichungstag der Anmeldung: 26.07.2006
(73) Patentinhaber: Möller Medical GmbH & Co.KG, 36043 Fulda (DE)
(72) Erfinder: Herget, Bernhard, 36115 Ehrenberg (DE); Schäfer, Siegfried, 36100 Petersberg (DE); Knacker, Peter, 36115 Ehrenberg (DE)
(74) Vertreter: Grünecker, Kinkeldey, Stockmair & Schwanhäusser Anwaltssozietät

(56) Entgegenhaltungen:
- EP-A- 1 293 228
- US-A- 4 668 217
- US-A- 4 997 421
- US-A- 6 056 718
- US-B1- 6 171 281

## Beschreibung

Die Erfindung betrifft ein Kanülenansatzsystem mit den Merkmalen des Oberbegriffs des Anspruchs 1.

Derartige Kanülenansatzsysteme finden in der Medizin Verwendung. Sie dienen dazu z.B. eine Kanüle konzentrisch in einem Hohlkörper festzulegen, wobei durch die Ansatzstücke die relative Lage der Instrumente zueinander bestimmt wird.

Bei bekannten Kanülenansatzsystemen dieser Gattung wird die Position eines Kanülenansatzes gegenüber einem zweiten Ansatzstück durch eine Übergangspassung festgelegt. Dabei wird eine das Instrument tragende, in Einführungsrichtung konisch zulaufende Anformung am vorderen Ende des zweiten Ansatzstückes in eine rohrartige Anformung am hinteren Ende des ersten Ansatzstückes eingeführt. Da der Innendurchmesser der rohrartigen Anformung geringfügig kleiner als der Außendurchmesser an der Basis der konischen Anformung ist, wird die konische Anformung des zweiten Ansatzstücks bei vollständigem Ineinanderschieben der Ansatzstücke in der rohrartigen Anformung des ersten Ansatzstücks leicht geklemmt, wodurch eine lösbare aber dennoch feste Positionierung der Ansätze erreicht werden soll.

In der Praxis bietet diese Verbindung jedoch oft nicht den nötigen Halt, da die Übergangspassung zu leichtgängig und damit als Spielpassung ausfällt. Umgekehrt kann die Übergangspassung auch zu eng ausfallen, so dass ein vollständiges Ineinanderschieben der Ansatzstücke nicht mehr möglich ist. Dadurch erreichen auch die Instrumentenspitzen ihre angestrebte Endposition relativ zueinander nicht. Um den Innendurchmesser der rohrartigen Anformung so auf den Außendurchmesser der konischen Anformung abzustimmen, dass beim Zusammenführen eine feste Verbindung beider Ansatzstücke erfolgt, welche jedoch auch ohne großen Aufwand wieder zu lösen ist, sind enge Toleranzen nötig. Wollte man gewährleisten, eine solch difiziele Passung jeweils mit der gleichen Passgenauigkeit zu fertigen, müsste der eigentlichen Fertigung ein gesonderter Ausleseprozess nachgeschaltet werden, in welchem die Passung aus Einzelteilen zusammengepasst wird. Daraus ist erkennbar, dass eine derart präzise Passung selbst durch enge Toleranzen schwer herstellbar ist und somit einen erhöhten Aufwand und damit erhöhte Produktionskosten bedeutet. Ferner ist das hier angesprochene Kanülenansatzsystem in der Regel in Kunststoff ausgeführt wird, was die Passgenauigkeit bei dünnwandigen Elementen mit sehr geringen Toleranzen zusätzlich erschwert, da gerade Kunststoffteile einem Verzug in Folge thermischer Einflüsse sowie einer leichten Verformbarkeit in Folge hoher Belastungen unterliegen. So können sich z.B. Übergangspassungen bei öfterem Benutzen weiten. Außerdem sind die Widerstände, die eine derartige Passung bietet, keineswegs für alle Bewegungsrichtungen gleich. So bietet eine Übergangspassung, die in axialer Richtung noch genügend Halt bietet, unter Umständen keine ausreichende Verdrehsicherheit.

In der US 6,056,718 wird ein Infusionsset beschrieben, bei welchem ein Infusionsansatz über Rastarme mit einem Kanülenansatz verbindbar ist. Die Rastarme des Infusionsansatzes rasten beim Zusammenschieben der beiden Ansatzstücke in entsprechende Ausnehmungen des Kanülenansatzes ein und sichern die Ansatzstücke somit gegen axiales Lösen. Durch die flache, nicht rotationssymmetrische Ausführung der beiden Ansatzstücke gewährleisten die Rastarme gleichzeitig eine Verdrehsicherheit der Ansatzstücke gegeneinander.

Ein in der US 4,997,421 gezeigtes Ansatzsystem besteht aus einem Infusionsansatz, einem Katheteransatz und einem Stabilisierungselement. Der Infusionsansatz weist an seinem vorderen Ende eine konische Anformung auf, welche mit einer zylindrischen Öffnung des Katheteransatzes eine Übergangspassung bildet. Die so gekoppelten Ansatzstücke werden anschließend in das Stabilisierungselement eingeschoben, welches ein axiales Lösen der Übergangspassung verhindern soll.

Die US 6,171,281 beschreibt ein Katheterverbindungssystem, bei welchem die Ansatzstücke miteinander verschraubt werden. Zudem weist eines der Ansatzstücke Vorsprünge auf, welche gegen Ende des Einschraubprozesses in entsprechenden Ausnehmungen an dem anderen Ansatzstück eingreifen.

Der Erfindung liegt die Aufgabe zugrunde ein Kanülenansatzsystem der eingangs genannten Gattung dahingehend zu verbessern, dass die Ansatzstücke für den Gebrauch möglichst einfach und doch ausreichend genau zueinander positionierbar sind, wobei sie mit mäßigen Toleranzen herstellbar sind.

Diese Aufgabe wird erfindungsgemäß gelöst mit einem Kanülenansatzsystem mit den Merkmalen des Anspruchs 1.

Durch den Formschluss werden die Ansatzstücke in ihrer Winkelposition zueinander festgelegt. In diesem formschlüssig gekoppelten Zustand können die Ansatzstücke mit Hilfe eines Verbindungselements fixiert werden. Zusätzlich werden die Ansatzstücke in ihrer axialen Lage zueinander fixiert. Durch die mehrteilige Ausführung können, im Gegensatz zur einteiligen Ausführung, größere Toleranzen gewählt werden, ohne dass der Wirkmechanismus beeinflusst wird. Dadurch lassen sich auch die Produktionskosten niedrig halten. Die Handhabung ist von einer Bedienperson ohne Schwierigkeiten durchzuführen.

In einer vorteilhaften Ausführung der Erfindung können beide Ansatzstücke zur Erzeugung des Formschlusses jeweils mindestens ein Positionierelement aufweisen. Durch die korrespondierenden Positionierelemente wird beim Zusammenführen der Ansatzstücke eine Formschlüssigkeit erreicht, wodurch die Winkelposition der Ansatzstücke relativ zueinander festgelegt wird. Vorteilhafterweise kann das eine Ansatzstück an seiner dem zweiten Ansatzstück zugewandeten Seite mindestens eine Positioniernut aufweisen, welche mit einem Positionierabsatz des zweiten Ansatzstücks in Eingriff gebracht werden kann, der sich an der dem ersten Ansatzstück zugewandten Seite befindet. Die Positioniernut ermöglicht zusammen mit dem Positionierabsatz ein gutes, leichtgängiges Zusammenfinden der Positionierelemente beim axialen Zusammenführen der beiden Ansatzstücke.

In einer weiteren günstigen Ausführungsform der Erfindung kann das erste Ansatzstück an seiner dem zweiten Ansatzstück zugewandten Seite eine rohrartige Anformung mit einem Flansch aufweisen, welcher mit mindestens einer radialen Ausformung versehen ist, an welcher ein Positionierelement angebracht ist, und an einer dem ersten Ansatzstück zugewandten Seite des zweiten Ansatzstücks ein korrespondierendes Positionierelement angebracht sein. Durch die Anbringung der Positionierelemente auf der radialen Ausformung, wie sie häufig zur Sicherung von Luer-Lock-Verbindungen Verwendung findet, bzw. an der Seite des Ansatzstücks lässt sich die Form der bisherigen, bekannten Ansatzstücke durch leichte Modifikation weiter verwenden. Dies reduziert die Herstellkosten und erhöht die Kompatibilität.

In einer besonders vorteilhaften Ausführung der Erfindung können an einem Ansatzstück mehrere Positionierelemente vorgesehen sein, welche jeweils mit Positionierelementen des zweiten Ansatzstücks korrespondieren, wobei mindestens jeweils zwei der Positionierelemente eine zu den anderen, gleichartigen Positionierelementen unterschiedliche Lage, Größe und/oder Form aufweisen. Durch die unterschiedliche Ausbildung bzw. Anordnung von mindestens zwei Positionierelementen, lässt sich zusammen mit den korrespondierenden Positionierelementen eine exakte Winkelposition der Ansatzstücke gegeneinander festlegen.

Bei einer weiteren Ausführungsform der Erfindung können sich die Positionierelemente jeweils an den einander zugewandten Stirnflächen der Ansatzstücke befinden. Dadurch wird ein besonders leichtes, axiales Zusammenfinden der Positionierelemente in Zusammenführrichtung der Ansatzstücke gewährleistet.

Bei einem Ausführungsbeispiel der Erfindung kann das Verbindungselement eine Ausnehmung aufweisen, welche sich einer rohrartigen Anformung an der dem zweiten Ansatzstück zugewandten Seite des ersten Ansatzstücks anpasst, wobei die radiale Ausdehnung der Ausnehmung größer als der Radius der rohrartigen Anformung, jedoch kleiner als der größte durch eine an der rohrartigen Anformung vorgesehene radiale Ausformung vorgegebene Radius ist. Damit sich das Verbindungselement beim Aufschieben auf die formschlüssig gekoppelten Ansatzstücke der rohrartigen Anformung gut anpasst, ist die radiale Ausdehnung der Ausnehmung etwas größer als der Radius der rohrartigen Anformung. Um jedoch die radialen Ausformungen am vorderen Ende der rohrartigen Anformung hintergreifen zu können, ist die radiale Ausdehnung der Ausnehmung kleiner als die radiale Ausdehnung der radialen Anformung. Durch diesen Hintergriff ist sichergestellt, dass die radialen Anformungen im Inneren des Verbindungselements eingeschlossen werden.

In einem günstigen Ausführungsbeispiel der Erfindung kann das Verbindungselement in seiner Endposition jeweils einen Teil der Ansatzstücke umfassen. Damit werden die Ansatzstücke in dem gekoppelten, formschlüssigen Zustand axial festgelegt. Es werden sowohl die Ansatzstücke in axialer Richtung relativ zueinander fixiert als auch die formschlüssige Verbindung gesichert, die die Winkelposition der Ansatzstücke zueinander festlegt,.

Bei einer vorteilhaften Ausführung der Erfindung kann das Verbindungselement an einer Wandung eine Ausnehmung aufweisen, welche ein Hintergreifen der Stirnfläche des Ansatzstückes sowie ein gleichzeitiges seitliches Umfassen des Ansatzstückes durch das Verbindungselement erlaubt. Nach dem Anbringen des Verbindungselements wird die Stirnfläche des ersten Ansatzstücks von der einen Wandung des Verbindungselements hintergriffen und kann so mit dem zweiten Ansatzstück, welches durch die andere Wandung des Verbindungselements hintergriffen wird, im Inneren des Verbindungselements zusammengehalten werden.

In einer weiteren Ausführungsform der Erfindung kann der lichte Abstand von Innenflächen des Verbindungselements nur geringfügig größer als oder etwa gleich der Summe der Dicken von umfassten Wandungen der beiden Ansatzstücke sein. Über den lichten Abstand der Innenflächen lässt sich die Pressung bestimmen, die auf die beiden im Inneren des Verbindungselementes zusammengefassten Flächen der beiden Ansatzstücke wirkt. Um die in diesem Fall beabsichtigte axiale Fixierung der Ansatzstücke relativ zueinander bzw. die Sicherung der formschlüssigen Verbindung im Inneren des Verbindungselements zu erhalten, müssen die die Positionierelemente tragenden Flächen nicht zwangsläufig mit Hilfe einer Übergangspassung gegeneinander gepresst werden. Je nach Abmessung der Positionierelemente ist hier auch eine leichte Spielpassung möglich, was dementsprechend große Toleranzen sowohl für den lichten Abstand der Innenflächen des Verbindungselements als auch für die Maße der Positionierelemente erlaubt.

Vorteilhafterweise kann das Verbindungselement etwa quer zu der Richtung aufgebracht werden, in welcher der Formschluss zustande kommt. Hierdurch wirkt die durch die Wandungen des Verbindungselements auf die Ansatzstücke aufgebrachte Kraft quer zu der Zusammenführrichtung der Ansatzstücke, durch welche der Formschluss erzeugt wird, und sichert diese so gegen Lösen.

In einem günstigen Ausführungsbeispiel kann das Ansatzsystem eine Griffplatte aufweisen, welche an mindestens einem der Ansätze lösbar befestigbar ist, wobei die Griffplatte eine Ausnehmung aufweist, an welche zwei Stifte grenzen, die in Ausnehmungen an einem Zentralkörper eines Ansatzstückes lösbar einsteckbar sind. Die Möglichkeit eine Griffplatte anzubringen, erleichtert die Handhabung des gesamten Kanülenansatzsystems in der Praxis wesentlich. Durch die spezielle Form der Ausnehmung mit den beiden Stiften, passt sich die Griffplatte dem Grundkörper des Ansatzstückes optimal an und kann so am vorderen Ende des Ansatzstückes einfach aufgesteckt werden.

Bei einer vorteilhaften Ausführung kann wahlweise eine Griffplatte oder das Verbindungselement an etwa der gleichen Stelle eines Ansatzstückes anbringbar sein. Dadurch ist ein Aneinanderreihen mehrerer Ansatzstücke, wie sie für den jeweiligen Einsatzfall benötigt werden, möglich. Zusätzlich kann die Griffplatte an dem Ansatzstück angebracht werden, an welchem die Stelle noch nicht durch ein Verbindungselement belegt ist, so dass eine gute Handhabbarkeit des Ansatzsystems gewährleistet ist.

Vorteilhafterweise kann mindestens eines der Ansatzstücke als Mandrin- oder Kanülenansatz ausgebildet sein. Dadurch lässt sich ein Kanülenansatz sowohl mit einem Mandrin- als auch einem weiteren Kanülenansatz gut und sicher verbinden.

Im Folgenden wird ein Ausführungsbeispiel der Erfindung anhand der folgenden Zeichnungen beschrieben. Darin zeigen:
- Figur 1: ein erfindungsgemäßes Kanülenansatzsystem mit zwei voneinander beabstandeten Ansatzstücken, wobei das zweite Ansatzstück ebenfalls als Kanülenansatz ausgeführt ist, einem Verbindungselement und einer Griffplatte,
- Figur 2: ein erfindungsgemäßes Kanülenansatzsystem mit zwei ineinander geführten, durch Formschluss in ihrer Winkelposition zueinander festgelegten Ansatzstücken, einem Verbindungselement und einer Griffplatte,
- Figur 3: ein erfindungsgemäßen Kanülenansatzsystem mit zwei ineinander geführten Ansatzstücken, welche durch ein Verbindungselement axial miteinander verbunden sind, und aufgesteckter Griffplatte und
- Figur 4: den vorderen Teil eines Ansatzstücks von oben gesehen.

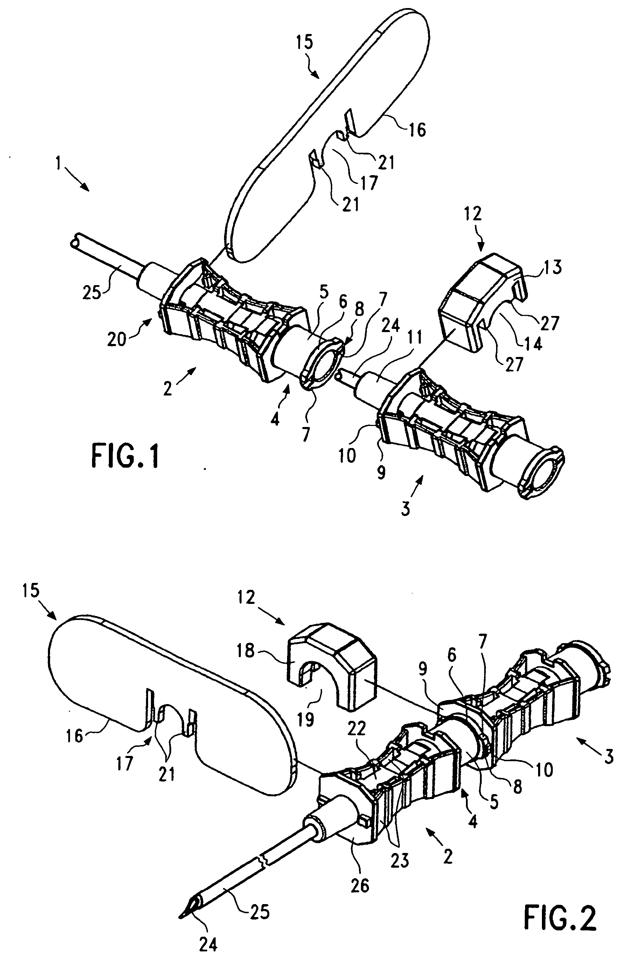

Figur 1 zeigt ein erfindungsgemäßes Kanülenansatzsystem 1 mit zwei gleichartigen Ansatzstücken 2, 3. Das Kanülenansatzsystem 1 kann jedoch auch aus zwei ungleichen Ansatzstücken z.B. einem Kanülenansatz und einem Mandrinansatz bestehen. Das erste Ansatzstück 2 trägt an seinem vorderen Ende 20 einen Hohlkörper 25 und ist hinter dem zweiten Ansatzstück 3, welches an seinem vorderen Ende eine Kanüle 24 trägt, axial beabstandet angeordnet. Durch axiales Zusammenführen der Ansatzstücke wird die Kanüle 24 konzentrisch in den Hohlkörper 25 eingeführt.

Das steckseitige Ende 4 des ersten Ansatzstücks 2 ist als weibliches Teil einer Luer-Lock-Verbindung ausgebildet. Dabei weist das Ende einer rohrartigen Anformung 5 einen flanschartig ausgebildeten Rand 6 mit zwei diametral angeordneten, radialen Anformungen 7 auf. Die radialen Anformungen 7 sind mit unterschiedlich breiten, radial verlaufenden Positioniernuten 8 versehen.

Eine steckseitige Stirnseite 9 des zweiten Ansatzstückes 3 weist zwei unterschiedlich breite, diametral angeordnete Positionierabsätze 10 auf, welche mit den Positioniemuten 8 korrespondieren. Die Positionierabsätze10 sind in Figur 2 an einer zur steckseitigen Stirnseite 9 analogen Fläche 26 erkennbar.

Ausgehend von der steckseitigen Stirnseite 9 erstreckt sich ein Konus 11, welcher sich mit zunehmendem Abstand zur Stirnseite 9 verjüngt und die Kanüle 24 beinhaltet.

Die seitlichen Flächen 23 der Ansatzstücke 2, 3 sind mit Rippen versehen. Zusätzlich können die Seitenflächen 23 wie hier leicht nach innen durchgebogen sein. So entstehen komfortable und sicherere Halteflächen.

Seitlich beabstandet zu dem zweiten Ansatzstück 3 befindet sich ein Verbindungselement 12, dessen hintere Wandung 13 eine Ausnehmung 14 aufweist. Die Ausnehmung 14 ist dabei so geformt, dass zwei Stifte 27 entstehen.

Seitlich beabstandet zu dem ersten Ansatzstück 2 ist eine Griffplatte 15 zu sehen, in deren unterem Rand 16 sich eine mittige Ausnehmung 17 mit zwei Stifte 21 befindet.

Figur 2 zeigt das Kanülenansatzsystem 1, bei welchem das zweite Ansatzstück 3 in das erste Ansatzstück 2 eingeführt ist. Die in Figur 1 verwendeten Bezugszeichen bezeichnen dieselben Teile wie in Figur 2, so dass diesbezüglich auf die Beschreibung von Figur 1 verwiesen wird.

In diesem, zusammengeführten Zustand ist der Konus 11, welcher das männliche Teil eines Luer-Locks repräsentiert, vollständig in die rohrartige Ausformung 5 des ersten Ansatzstückes 2, welche den weiblichen Teil eines Luer-Locks darstellt, eingeführt. Dadurch liegt der flanschartig ausgebildete Rand 6 mit seinen radialen Ausformungen 7 direkt an der steckseitigen Stirnseite 9 des zweiten Ansatzstücks 3 an, wobei die Positionierabsätze 10 in die Positioniemuten 8 eingreifen.

Seitlich beabstandet zu den verbundenen Ansatzstücken befindet sich das Verbindungselement 12, dessen vordere Wandung 18 eine runde, etwa halbkreisförmige Öffnung 19 aufweist.

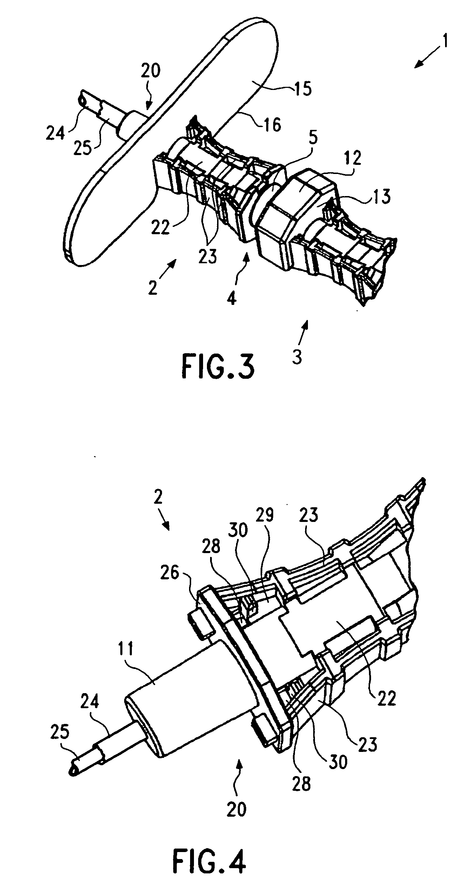

Figur 3 zeigt das Kanülenansatzsystem 1, bei welchem die Ansatzstücke 2 und 3 durch das Verbindungselement 12 verbunden sind. Die in den Figuren 1 und 2 verwendeten Bezugszeichen bezeichnen dieselben Teile wie in Figur 3, so dass diesbezüglich auf die Beschreibungen der Figuren 1 und 2 verwiesen wird.

Das Verbindungselement 12 ist dabei quer zur Axialrichtung der Kanüle 24 auf die formschlüssig ineinander greifenden Ansatzstück 2 und 3 aufgesteckt. Seine hintere Wandung 13 hintergreift dabei die steckseitige Stirnseite 9 des zweiten Ansatzstücks 3 und seine vordere Wandung 18 hintergreift die radialen Ausformungen 7 des flanschartig ausgebildeten Rands 6 des ersten Ansatzstücks 2.

Die Griffplatte 15 ist am vorderen, dem steckseitigen Ende 4 gegenüberliegenden Ende 20 des ersten Ansatzstücks 2 mittels der beiden Stifte 21 befestigt, wobei die Stifte 21 in Ausnehmungen 28 am Zentralkörper 22 des Ansatzstücks eingesteckt sind. Die Ausnehmungen 28 sind in Figur 4 erkennbar. Durch die Haftreibung der Stifte 21 in den Ausnehmungen 28 wird die Griffplatte 15 in ihrer Position fixiert. Um ein ungewolltes Lösen der Griffplatte 15 zu verhindern, können die Stifte 21 auch als Rasten ausgeführt sein.

Figur 4 zeigt den vorderen Teil des Ansatzstücks 2 von oben gesehen in vergrößerter Form. Die in den Figuren 1 bis 3 verwendeten Bezugszeichen bezeichnen dieselben Teile wie in Figur 4, so dass diesbezüglich auf die Beschreibung der Figuren 1 bis 3 verwiesen wird.

Das Ansatzstück 2 weist an seinem vorderen Ende 20 direkt hinter der zur steckseitigen Stirnseite 9 analogen Fläche 26 zwei Ausnehmungen 28 auf. Diese korrespondieren in ihrer Form mit den Stiften 21 der Griffplatte 15 bzw. den Stiften 27 des Verbindungselements 12 und befinden sich in einer horizontalen Mittelfläche 29 des Zentralkörpers 22. Unmittelbar hinter den Ausnehmungen 28 befinden sich zwei Rippen 30. Diese erstrecken sich von den seitlichen Flächen 23 des Ansatzstücks nach innen. So sind die Stifte 21 bzw. 27 beim Aufschieben der Griffplatte 15 bzw. des Verbindungselements 12 gut geführt.

Im Folgenden wird die Funktionsweise des in den Figuren dargestellten, erfindungsgemäßen Ausführungsbeispiels erläutert.

Durch axiales Zusammenführen der beiden Ansatzstücke 2 und 3 wird der Konus 11 des zweiten Ansatzstücks 3 in die rohrartige Anformung 5 des ersten Ansatzstücks 2 eingeführt bis der flanschartige Rand 6 mit den radialen Ausformungen 7 auf der steckseitigen Stirnseite 9 des zweiten Ansatzstücks 3 zu liegen kommt. Dabei finden die Positionierabsätze 10, welche sich auf der steckseitigen Stirnseite 9 befinden, in die Positioniernuten 8 und stellen somit einen Formschluss her, der die Winkelposition des ersten Ansatzstücks 2 gegenüber dem zweiten Ansatzstück 3 exakt festlegt.

Durch das Aufschieben des Verbindungselements, welches die steckseitige Stirnfläche 9 des zweiten Ansatzstücks 3 und die radialen Ausformungen 7 am flanschartig ausgebildeten Rand 6 des ersten Ansatzstücks 2 hintergreift, werden diese Bauteile und damit auch die Ansatzstücke 2 und 3 axial zusammengehalten. Da sich die Positionierelemente in Form der -nuten und -absätze, welche den Formschluss bewirken, ebenfalls auf diesen Bauteilen befinden, wird durch das Verbindungselement auch die formschlüssige Verbindung gegen Lösen gesichert. Die spezielle Form der Ausnehmung 14 bzw. der halbkreisförmigen Öffnung 19 in den Wandungen 13, 18 des Verbindungselements 12 ermöglichen dabei das exakte Hintergreifen bzw. die Anpassung an die jeweilige Geometrie der Ansatzstücke.

Der lichte Innenabstand der Wandungen 13, 18 kann dabei so eng gewählt werden, dass das Verbindungselement 12 beim Aufschieben mit einer leichten Übergangs- bzw. Presspassung auf den eingeschlossenen Flächen sitzt. Hierdurch wird eine reibschlüssige Sicherung des Verbindungselements 12 erreicht. Zusätzlich ist das Verbindungselement 12 über die in seiner hinteren Wandung 13 ausgebildeten Stifte 27 mit dem Ansatzstück 3 verbunden. Die Stifte 27 greifen dabei in die Ausnehmungen 28 in der Mittelfläche 29 des Zentralkörpers 22 ein. Die Haftreibung der Stifte 21 in den Ausnehmungen 28 fixiert das Verbindungselement 12. Durch diese zusätzliche Sicherung des Verbindungselements 12 ist es auch möglich, den lichten inneren Abstand der Wandungen 13, 18 größer zu wählen, so dass keine Press- sondern eine leichte Spielpassung entsteht. Das Spiel, welches sich in der formschlüssigen Verbindung aus Positioniemuten 8 und Positionierabsätzen 10 einstellt, ist dabei von dem lichten Innenabstand der Wandungen 13 und 18 abhängig.

Mit diesem Wirkprinzip lassen sich mehrere Ansatzstücke, welche jeweils ein anderes Instrument tragen können, aneinanderreihen, um so ein auf den jeweiligen Einsatzfall individuell abgestimmtes Instrument bzw. Instrumentsystem zu erhalten.

Da die mittige Ausnehmung 17 im unteren Rand 16 der Griffplatte 15 ähnlich geformt ist wie die Ausnehmung 14 in der hinteren Wandung 13 des Verbindungselements 12, lässt sich die Griffplatte 15 an einer beliebigen, freien Stelle, an welcher noch kein Verbindungselement 14 angebracht ist, hinter einer zur steckseitigen Stirnseite 9 analogen Stirnseite anbringen. Die beiden Stifte 21 werden dabei in die Ausnehmungen 28 des Zentralkörpers 22 gesteckt, wo sie durch Haftreibung fixiert sind.

## Patentansprüche

1. Kanülenansatzsystem (1) für den medizinischen Einsatz, mit mindestens zwei Ansatzstücken (2, 3), welche entlang einer jeweiligen längsachse hintereinander anordbar und miteinander koppelbar sind, wobei die Ansatzstücke (2, 3) durch Formschluss beim axialen Zusammenführen lösbar und verdrehsicher miteinander verbindbar sind und in diesem Zustand axial lösbar fixierbar sind,
**dadurch gekennzeichnet,**
**dass** die axiale Fixierung durch ein separat anbringbares Verbindungselement (12) erfolgt, das quer zu der Richtung aufgesteckt wird, in welcher der Formschluss zustande kommt.

2. Kanülenansatzsystem (1) nach Anspruch 1,
**dadurch gekennzeichnet,**
**dass** beide Ansatzstücke zur Erzeugung des Formschlusses jeweils mindestens ein Positionierelement aufweisen.

3. Kanülenansatzsystem (1) nach einem der vorhergehenden Ansprüche,
**dadurch gekennzeichnet,**
**dass** das eine Ansatzstück (2) an seiner dem zweiten Ansatzstück (3) zugewandten Seite als Positionierelement mindestens eine Positioniemut (8) aufweist, welche mit einem Positionierabsatz (10) des zweiten Ansatzstücks in Eingriff gebracht werden kann, der sich an der dem ersten Ansatzstück zugewandten Seite befindet.

4. Kanülenansatzsystem (1) nach Anspruch 2 oder 3,
**dadurch gekennzeichnet,**
**dass** das erste Ansatzstück (2) an seiner dem zweiten Ansatzstück (3) zugewandten Seite eine rohrartige Anformung (5) mit einem Flansch (6) aufweist, welcher mit mindestens einer radialen Ausformung (7) versehen ist, an welcher ein Positionierelement angebracht ist und, dass an einer dem ersten Ansatzstück (2) zugewandten Seite des zweiten Ansatzstücks (3) ein korrespondierendes Positionierelement angebracht ist.

5. Kanülenansatzsystem (1) nach einem der vorhergehenden Ansprüche,
**dadurch gekennzeichnet,**
**dass** an einem Ansatzstück (2) mehrere Positionierelemente vorgesehen sind, welche jeweils mit Positionierelementen des zweiten Ansatzstücks (3) korrespondieren, wobei mindestens jeweils zwei der Positionierelemente eine zu den anderen, gleichartigen Positionierelementen unterschiedliche Lage, Größe und/oder Form aufweisen.

6. Kanülenansatzsystem (1) nach eimem der Ansprüche 2-5,
**dadurch gekennzeichnet,**
**dass** sich die Positionierelemente jeweils an den einander zugewandten Stirnflächen der Ansatzstücke (2, 3) befinden.

7. Kanülenansatzsystem (1) nach einem der Ansprüche 4-6,
**dadurch gekennzeichnet,**
**dass** das Verbindungselement (12) eine Ausnehmung (19) aufweist, welche etwa einer rohrartigen Anformung (5) an der dem zweiten Ansatzstück (3) zugewandten Seite des ersten Ansatzstücks (3) angepasst ist, wobei die radiale Ausdehnung der Ausnehmung (19) größer als der Radius der rohrartigen Anformung (5) jedoch kleiner als der größte durch eine an der rohrartigen Anformung (5) vorgesehene radiale Ausformung (7) vorgegebene Radius ist.

8. Kanülenansatzsystem (1) nach einem der vorhergehenden Ansprüche,
**dadurch gekennzeichnet,**
**dass** das Verbindungselement (12) in seiner Endposition jeweils einen Teil der Ansatzstücke umfasst.

9. Kanülenansatzsystem (1) nach einem der vorhergehenden Ansprüche,
**dadurch gekennzeichnet,**
**dass** das Verbindungselement (12) an einer Wandung (13) eine Ausnehmung aufweist, welche ein Hintergreifen der Stirnfläche des Ansatzstücks sowie ein gleichzeitiges seitliches Umfassen des Ansatzstücks durch das Verbindungselement erlaubt.

10. Kanülenansatzsystem (1) nach einem der vorhergehenden Ansprüche,
**dadurch gekennzeichnet,**
**dass** der lichte Abstand von Innenflächen des Verbindungselements (12) nur geringfügig größer als oder etwa gleich der Summe der Dicken von umfassten Wandungen der beiden Ansatzstücke ist.

11. Kanülenansatzsystem (1) nach einem der vorhergehenden Ansprüche, wobei das Ansatzsystem eine Griffplatte (15) aufweist, welche an mindestens einem der Ansätze lösbar befestigbar ist, wobei die Griffplatte (15) eine Ausnehmung (17) aufweist,
**dadurch gekennzeichnet,**
**dass** an die Ausnehmung (17) zwei Stifte (21) grenzen, die in Ausnehmungen (28) an einem Zentralkörper (22) eines Ansatzstücks lösbar einsteckbar sind.

12. Kanülenansatzsystem (1) nach einem der vorhergehenden Ansprüche,
**dadurch gekennzeichnet,**
**dass** wahlweise eine Griffplatte (15) oder das Verbindungselement (12) an etwa der gleichen Stelle eines Ansatzstücks anbringbar sind.

13. Kanülenansatzsystem (1) nach einem der vorhergehenden Ansprüche,
**dadurch gekennzeichnet,**
**dass** mindestens eines der Ansatzstücke als Mandrin- oder Kanülenansatz ausgebildet ist.

## Claims

1. A cannula hub system (1) for medical use comprising at least two hubs (2, 3) which are adapted to be arranged one after the other and coupled to one another along a respective longitudinal axis, said hubs (2, 3) being positively engageable, when they are combined in the axial direction, and connectable to one another such that they are releasable and secured against rotation relative to one another, and they are adapted to be fixed in an axially releasable manner in this condition,
**characterized in that**
said axial fixing is effected by a separately attachable connection element (12) which is attached transversely to the direction in which the positive engagement is established.

2. A cannula hub system (1) according to claim 1,
**characterized in that,**
for establishing the positive engagement, the two hubs are each provided with at least one positioning element.

3. A cannula hub system (1) according to one of the preceding claims,
**characterized in that**
the first hub (2) has, on the side facing the second hub (3), at least one positioning groove (8) used as a positioning element, said positioning groove (8) being adapted to be brought into engagement with a positioning shoulder (10) of the second hub provided on the side facing the first hub.

4. A cannula hub system (1) according to claim 2 or 3,
**characterized in that**
the side of the first hub (2) facing the second hub (3) has formed thereon a tubular piece (5) provided with a flange (6) which is provided with at least one radial bulge (7) having attached thereto a positioning element, and that a corresponding positioning element is attached to the side of the second hub (3) facing the first hub (2).

5. A cannula hub system (1) according to one of the preceding claims,
**characterized in that**
the first hub (2) has provided hereon a plurality of positioning elements which each correspond to positioning elements of the second hub (3), at least two respective ones of said positioning elements having a position, size and/or shape which differs from that of the other, similar positioning elements.

6. A cannula hub system (1) according to one of the claims 2 to 5,
**characterized in that**
the respective positioning elements are located on the end faces of the hubs (2, 3) which face one another.

7. A cannula hub system (1) according to one of the claims 4 to 6,
**characterized in that**
the connection element (12) is provided with an opening (19) which is approximately adapted to a tubular piece (5) formed on side of the first hub (2) facing the second hub (3), the radial dimensions of the opening (19) being larger than the radius of said tubular piece (5) but smaller than the maximum radius defined by a radial bulge (7) provided on said tubular piece (5).

8. A cannula hub system (1) according to one of the preceding claims,
**characterized in that**
when occupying its end position, the connection element (12) encompasses a respective part of each hub.

9. A cannula hub system (1) according to one of the preceding claims,
**characterized in that**
the connection element (12) includes in a wall (13) an opening which allows said connection element (12) to engage behind the end face of the hub as well as to encompass the hub on the sides thereof.

10. A cannula hub system (1) according to one of the preceding claims,
**characterized in that**
the interior distance of inner surfaces of the connection element (12) is only slightly larger than or approximately equal to the sum of the thicknesses of encompassed walls of the two hubs.

11. A cannula hub system (1) according to one of the preceding claims, said hub system including a grip plate (15) which is adapted to be releasably secured to at least one of the hubs, said grip plate (15) having an opening (17),
**characterized in that**
two pins (21) adjoin the opening (17), said pins (21) being adapted to be releasably inserted in openings (28) on a central body (22) of a hub.

12. A cannula hub system (1) according to one of the preceding claims,
**characterized in that**
a grip plate (15) or the connection element (12) can selectively be arranged at approximately the same location of a hub.

13. A cannula hub system (1) according to one of the preceding claims,
**characterized in that**
at least one of the hubs is implemented as a stylet hub or a cannula hub.

## Revendications

1. Système d'embase de canule (1) à usage médical, avec au moins deux pièces d'embase (2, 3) qui peuvent être agencées l'une derrière l'autre le long d'un axe longitudinal respectif et qui peuvent être couplées l'une à l'autre, les pièces d'embase (2, 3) pouvant être assemblées l'une à l'autre par concordance de forme, amovibles et fixes en rotation, lors du rapprochement axial, et pouvant être fixées, amovibles axialement, dans cet état,
**caractérisé par le fait que**
la fixation axiale s'effectue par un élément de liaison (12) qui peut être amené séparément et qui est emboîté transversalement par rapport à la direction dans laquelle la concordance de forme est réalisée.

2. Système d'embase de canule (1) selon la revendication 1,
**caractérisé par le fait que**
les deux pièces d'embase comportent chacune, pour la mise en oeuvre la concordance de forme, au moins un élément de positionnement.

3. Système d'embase de canule (1) selon l'une des revendications précédentes,
**caractérisé par le fait que**
la première pièce d'embase (2) comporte comme élément de positionnement, sur son côté proche de la deuxième pièce d'embase (3), au moins une rainure de positionnement (8) qui peut être amenée en prise avec une partie en saillie de positionnement (10) de la deuxième pièce d'embase, laquelle partie en saillie se trouve sur le côté proche de la première pièce d'embase.

4. Système d'embase de canule (1) selon la revendication 2 ou 3,
**caractérisé par le fait que**
la première pièce d'embase (2) comporte, sur son côté proche de la deuxième pièce d'embase (3), une forme tubulaire (5) avec une bride (6) qui est munie d'au moins une jupe radiale (7) sur laquelle est placé un élément de positionnement, et un élément de positionnement correspondant est placé sur un côté, proche de la première pièce d'embase (2), de la deuxième pièce d'embase (3).

5. Système d'embase de canule (1) selon l'une des revendications précédentes,
**caractérisé par le fait que**
il est prévu sur une pièce d'embase (2) plusieurs éléments de positionnement qui correspondent à chaque fois à des éléments de positionnement de la deuxième pièce d'embase (3), au moins deux des éléments de positionnement comportant à chaque fois une position, dimension et/ou forme différente des autres éléments de positionnement du même type.

6. Système d'embase de canule (1) selon l'une des revendications 2 à 5,
**caractérisé par le fait que**
les éléments de positionnement se trouvent à chaque fois sur les surfaces frontales, proches l'une de l'autre, des pièces d'embase (2, 3).

7. Système d'embase de canule (1) selon l'une des revendications 4 à 6,
**caractérisé par le fait que**
l'élément de liaison (12) comporte un évidement (19) qui est sensiblement adapté à une forme tubulaire (5) sur le côté, proche de la deuxième pièce d'embase (3), de la première pièce d'embase (2), la dimension radiale de l'évidement (19) étant supérieure au rayon de la forme tubulaire (5) mais inférieure au plus grand rayon prescrit par une jupe radiale (7) prévue sur la forme tubulaire (5).

8. Système d'embase de canule (1) selon l'une des revendications précédentes,
**caractérisé par le fait que**
l'élément de liaison (12) entoure dans sa position finale à chaque fois une partie des pièces d'embase.

9. Système d'embase de canule (1) selon l'une des revendications précédentes,
**caractérisé par le fait que**
l'élément de liaison (12) comporte sur sa paroi (13) un évidement qui permet de saisir par derrière la surface frontale de la pièce d'embase et d'entourer en même temps latéralement la pièce d'embase avec l'élément de liaison.

10. Système d'embase de canule (1) selon l'une des revendications précédentes,
**caractérisé par le fait que**
l'écartement des surfaces intérieures de l'élément de liaison (12) est seulement légèrement supérieur ou environ égal à la somme des épaisseurs des parois entourées des deux pièces d'embase.

11. Système d'embase de canule (1) selon l'une des revendications précédentes, le système d'embase comportant une plaque de préhension (15) qui peut être fixée de manière amovible sur au moins l'une des embases, la plaque de préhension (15) comportant un évidement (17),
**caractérisé par le fait que**
deux broches (21) sont adjacentes à l'évidement (17), lesquelles broches peuvent être placées de manière amovible dans des évidements (28) sur un corps central (22) d'une pièce d'embase.

12. Système d'embase de canule (1) selon l'une des revendications précédentes,
**caractérisé par le fait que,**
au choix, une plaque de préhension (15) ou l'élément de liaison (12) peut être placé à peu près au même endroit qu'une pièce d'embase.

13. Système d'embase de canule (1) selon l'une des revendications précédentes,
**caractérisé par le fait que**
au moins l'une des pièces d'embase est conçue comme une embase de mandrin ou de canule.
